(19) Europäisches Patentamt / European Patent Office / Office européen des brevets

(11) **EP 2 709 682 B1**

(12) **EUROPÄISCHE PATENTSCHRIFT**

(45) Veröffentlichungstag und Bekanntmachung des Hinweises auf die Patenterteilung:
**14.12.2016 Patentblatt 2016/50**

(51) Int Cl.:
*A61L 15/26* *(2006.01)*      *A61L 15/42* *(2006.01)*
*A61L 15/60* *(2006.01)*

(21) Anmeldenummer: **12720872.6**

(22) Anmeldetag: **14.05.2012**

(86) Internationale Anmeldenummer:
**PCT/EP2012/058864**

(87) Internationale Veröffentlichungsnummer:
**WO 2012/156346 (22.11.2012 Gazette 2012/47)**

(54) **VERWENDUNG WASSERABSORBIERENDER POLYMERPARTIKEL ZUR ABSORPTION VON BLUT UND/ODER MENSTRUATIONSFLÜSSIGKEIT**

USE OF WATER-ABSORBING POLYMER PARTICLES FOR THE ABSORPTION OF BLOOD AND/OR MENSTRUAL FLUID

UTILISATION DE PARTICULES POLYMÈRES ABSORBANT L'EAU POUR ABSORBER LE SANG ET/OU LE LIQUIDE MENSTRUEL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.05.2011 US 201161487299 P**

(43) Veröffentlichungstag der Anmeldung:
**26.03.2014 Patentblatt 2014/13**

(73) Patentinhaber: **BASF SE**
**67056 Ludwigshafen am Rhein (DE)**

(72) Erfinder:
• **LOPEZ VILLANUEVA, Francisco Javier**
**67105 Schifferstadt (DE)**
• **LINSENBÜHLER, Markus**
**69121 Heidelberg (DE)**
• **WEISMANTEL, Matthias**
**63637 Jossgrund (DE)**
• **SIEGEL, Bernd**
**67166 Otterstadt (DE)**

(74) Vertreter: **BASF IP Association**
**BASF SE**
**ZRX-C6**
**67056 Ludwigshafen (DE)**

(56) Entgegenhaltungen:
WO-A1-2005/042042    WO-A1-2008/009598
WO-A1-2011/026876    WO-A1-2011/113728

EP 2 709 682 B1

**Beschreibung**

Die vorliegende Erfindung betrifft die Verwendung wasserabsorbierender Polymerpartikel zur Absorption von Blut und/oder Menstruationsflüssigkeit, wobei die wasserabsorbierenden Polymerpartikel durch Polymerisation einer aufgeschäumten Monomerlösung oder -suspension, Vortrocknen des polymeren Schaums auf einen Wassergehalt von 5 bis 30 Gew.-%, Mahlung des vorgetrockneten Schaums und Nachtrocknen auf den gewünschten Endwassergehalt erhältlich sind.

Wasserabsorbierende Polymere werden als wässrige Lösungen absorbierende Produkte zur Herstellung von Windeln, Tampons, Damenbinden, Slipeinlagen, Wundabdeckungen und anderen Hygieneartikeln, aber auch als wasserzurückhaltende Mittel im landwirtschaftlichen Gartenbau verwendet.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Wasserabsorbierende Schäume auf Basis von vernetzten Säuregruppen enthaltenden Monomeren sind bekannt, beispielsweise aus EP 0 858 478 B1, WO 97/31971 A1, WO 99/44648 A1 und WO 00/52087 A1. Sie werden beispielsweise durch Schäumen einer polymerisierbaren wässrigen Mischung, die zu mindestens 50 Mol-% neutralisierte, Säuregruppen enthaltende ethylenisch ungesättigte Monomere, Vernetzer und mindestens ein Tensid enthalten, und anschließendes Polymerisieren der geschäumten Mischung hergestellt. Das Schäumen der polymerisierbaren Mischung kann durch Dispergieren von feinen Blasen eines gegenüber Radikalen inerten Gases oder durch Lösen eines solchen Gases unter erhöhtem Druck in der polymerisierbaren Mischung und Entspannen der Mischung erfolgen. Die Schäume werden beispielsweise in Hygieneartikeln zur Akquisition, Distribution und Speicherung von Körperflüssigkeiten verwendet.

Wasserabsorbierende Polymerpartikel werden üblicherweise in Einwegwindeln zur Absorption von Urin eingesetzt und für diese Verwendung optimiert. Bei der Absorption wässriger Suspensionen können die wasserabsorbierenden Polymerpartikel zwar das in der wässrigen Suspension enthaltende Wasser absorbieren, nicht aber die in der Suspension enthaltenen ungelösten Feststoffe. Dies führt dazu, dass sich die Oberfläche der wasserabsorbierenden Polymerpartikel mit Feststoffpartikel belegt und der Zutritt von weiterem Wasser verhindert wird. Es hat daher nicht an Versuchen gefehlt wasserabsorbierende Polymerpartikel zur Absorption wässriger Flüssigkeiten aus Suspensionen wie Blut und Menstruationsflüssigkeit zu optimieren.

WO 2005/042042 A1 lehrt, dass wasserabsorbierender Polymerpartikel zur Verbesserung der Blutabsorption mit Tensiden und Alkoholen beschichtet werden.

Aufgabe der vorliegenden Erfindung war die Bereitstellung von Hygieneartikeln mit verbesserter Absorption von Blut und Menstruationsflüssigkeit.

Gelöst wurde die Aufgabe durch die Verwendung wasserabsorbierender Polymerpartikel zur Absorption von Blut und/oder Menstruationsflüssigkeit, wobei die wasserabsorbierenden Polymerpartikel durch Polymerisation einer aufgeschäumten wässrigen Monomerlösung oder - suspension, enthaltend

a) mindestens ein ethylenisch ungesättigtes, säuregruppentragendes Monomer, das zu 25 bis 95 mol-% neutralisiert ist,
b) mindestens einen Vernetzer,
c) mindestens einen Initiator,
d) mindestens ein Tensid,
e) optional ein oder mehrere mit den unter a) genannten Monomeren copolymerisierbare ethylenisch ungesättigte Monomere,
f) optional einen Lösevermittler und
g) optional Verdicker, Schaumstabilisatoren, Polymerisationsregler, Füllstoffe, Fasern und/oder Zellkeimbildner,

Vortrocknen des polymeren Schaums auf einen Wassergehalt von 5 bis 30 Gew.-%, Mahlung des vorgetrockneten Schaums und Nachtrocknen auf den gewünschten Endwassergehalt erhältlich sind.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydro-chinonmonomethylether.

US 6,107,358 A offenbart die Herstellung von wasserabsorbierenden Polymerpartikeln zur schnellen Absorption von Wasser, durch Aufschäumen der Monomerlösung durch inertes Gas und anschließende Polymerisation der Lösung.

Die Herstellung von wasserabsorbierenden Partikeln durch Einbringen eines festen Blähmittels in die zu polymerisierende Monomerlösung und anschließende Polymerisation wird in EP 0 744 435 A offenbart.

Die Menge an Monomer a) beträgt vorzugsweise 20 bis 90 Gew.-%, besonders bevorzugt 30 bis 85 Gew.-%, ganz besonders bevorzugt 35 bis 75 Gew.-%, jeweils bezogen auf das unneutralisierte Monomer a) und auf die Monomerlösung oder -suspension. Bezogen auf das unneutralisierte Monomer a) bedeutet im Rahmen dieser Erfindung, dass für die Berechnung der Anteil des Monomeren a) vor der Neutralisation verwendet wird, d.h. der Beitrag der Neutralisation bleibt unberücksichtigt.

Die Säuregruppen der Monomere a) sind zu 25 bis 95 mol-%, vorzugsweise zu 40 bis 85 mol-%, bevorzugt zu 50 bis 80 mol-%, besonders bevorzugt zu 55 bis 75 mol-%, neutralisiert, wobei die üblichen Neutralisationsmittel verwendet werden können, beispielsweise Alkalimetallhydroxide, Alkalimetalloxide, Alkalimetallcarbonate oder Alkalimetallhydrogencarbonate sowie deren Mischungen. Die Neutralisation kann jedoch auch mit Ammoniak, Aminen oder Alkanolaminen, wie Ethanolamin, Diethanolamin oder Triethanolamin, vorgenommen werden.

In einer bevorzugten Ausführungsform werden mindestens 50 mol-%, vorzugsweise mindestens 75 mol-%, besonders bevorzugt mindestens 90 mol-%, ganz besonders bevorzugt mindestens 95 mol-%, der neutralisierten Monomere a) mittels einer anorganischen Base, vorzugsweise Kaliumkarbonat, Natriumkarbonat oder Natriumhydroxid, neutralisiert wurden.

Ein hoher Neutralisationsgrad und ein hoher Anteil mit einer anorganischen Base neutralisierter Säuregruppen vermindert die Flexibilität der erhaltenen polymeren Schäume und erleichtert die anschließende Mahlung.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindesten 10 Gew.-ppm, besonders bevorzugt mindesten 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraalloxyethan, Methylenbismethacrylamid, Tetraallyammoniumchlorid, 15-fach ethoxiliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 1 bis 10 Gew.-%, besonders bevorzugt 2 bis 7 Gew.-%, ganz besonders bevorzugt 3 bis 5 Gew.-%, jeweils bezogen auf das unneutralisierte Monomer a). Mit steigendem Vernetzergehalt sinkt die Zentrifugenretentionskapazität (CRC) und die Absorption unter einem Druck von 21,0 g/cm$^2$ (AUL0.3psi) durchläuft ein Maximum.

Als Initiatoren c) können sämtliche unter den Polymerisationsbedingungen in Radikale erzeugende Verbindungen eingesetzt werden, beispielsweise thermische Initiatoren, Redox-Initiatoren, Photoinitiatoren.

Thermische Initiatoren sind beispielsweise Peroxide, Hydroperoxide, Wasserstoffperoxid, Persulfate und Azoinitiatoren. Geeignete Azoinitiatoren sind beispielsweise 2,2'-Azobis-(2-amidinopropan)dihydrochlorid, 2,2'-Azobis-(N,N-dimethylen)isobutyramidin-dihydrochlorid, 2-(Carbamoylazo)isobutyronitril, 2,2'-Azobis[2-(2'-imidazolin-2-yl)propan]dihydrochlorid und 4,4'-Azobis-(4-cyanovaleriansäure).

Photoinitiatoren sind beispielsweise $\alpha$-Spalter, H-abstrahierende Systeme und Azide. Geeignete $\alpha$-Spalter bzw. H-abstrahierende Systeme sind beispielsweise Benzophenon-Derivate, wie Michlers-Keton, Phenanthren-Derivate, Fluoren-Derivate, Anthrachinon-Derivate, Thioxanton-Derivate, Cumarin-Derivate, Benzoinether und deren Derivate, Azostarter, wie die oben genannten Radikalbildner, substituierte Hexaarylbisimidazole oder Acylphosphinoxide. Geeignete Azide sind beispielsweise 2-(N,N-Dimethylamino)-ethyl-4-azidocinnamat, 2-(N,N-Dimethylamino)-ethyl-4-azidonaphthylketon, 2-(N,N-Dimethylamino)-ethyl-4-azidobenzoat, 5-Azido-1-naphthyl-2'-(N,N-dimethylamino)ethylsulfon, N-(4-Sulfonylazidophenyl)maleinimid, N-Acetyl-4-sulfonylazidoanilin, 4-Sulfonylazidoanilin, 4-Azidoanilin, 4-Azidophenacylbromid, p-Azidobenzoesäure, 2,6-Bis(p-azidobenzyliden)cyclohexanon und 2,6-Bis-(p-azido-benzyliden)-4-methylcyclohexanon.

Die Initiatoren c) werden in üblichen Mengen eingesetzt, vorzugsweise mindestens 0,01 mol-%, besonders bevorzugt mindestens 0,05 mol-%, ganz besonders bevorzugt mindestens 1 mol-%, sowie üblicherweise weniger als 5 mol-%, vorzugsweise weniger als 2 mol-%, bezogen auf die Monomere a).

Die Tenside d) sind für die Herstellung und die Stabilisierung der aufgeschäumten Monomerlösung oder -suspension von entscheidender Bedeutung. Man kann anionische, kationische oder nichtionische Tenside oder Tensidmischungen verwenden, die miteinander verträglich sind. Man kann niedermolekulare oder auch polymere Tenside einsetzen, wobei sich Kombinationen unterschiedlicher oder auch gleichartiger Typen von Tensiden als vorteilhaft herausgestellt haben. Verwendbare nichtionische Tenside sind beispielsweise Additionsprodukte von Alkylenoxiden, insbesondere Ethylenoxid, Propylenoxid und/oder Butylenoxid an Alkohole, Amine, Phenole, Naphthole oder Carbonsäuren. Vorteilhaft setzt man als Tenside Additionsprodukte von Ethylenoxid und/oder Propylenoxid an mindestens 10 C-Atome enthaltende Alkohole ein, wobei die Additionsprodukte pro Mol Alkohol 3 bis 200 Mol Ethylenoxid und/oder Propylenoxid angelagert enthalten. Die Additionsprodukte enthalten die Alkylenoxid-Einheiten in Form von Blöcken oder in statistischer Verteilung. Beispiele für einsetzbare nichtionische Tenside sind die Additionsprodukte von 7 Mol Ethylenoxid an 1 Mol Talgfettalkohol, Umsetzungsprodukte von 9 Mol Ethylenoxid mit 1 Mol Talgfettalkohol und Additionsprodukte von 80 Mol Ethylenoxid an 1 Mol Talgfettalkohol. Weitere verwendbare handelsübliche nichtionische Tenside bestehen aus Umsetzungsprodukten von Oxoalkoholen oder Ziegler-Alkoholen mit 5 bis 12 Mol Ethylenoxid pro Mol Alkohol, insbesondere mit 7 Mol Ethylenoxid. Weitere verwendbare handelsübliche nichtionische Tenside werden durch Ethoxylierung von Rizinusöl erhalten. Pro Mol Rizinusöl werden beispielsweise 12 bis 80 Mol Ethylenoxid angelagert. Weitere einsetzbare handelsübliche Produkte sind beispielsweise die Umsetzungsprodukte von 18 Mol Ethylenoxid mit 1 Mol Talgfettalkohol, die Additionsprodukte von 10 Mol Ethylenoxid an 1 Mol eines $C_{13}/C_{15}$-Oxoalkohols, oder die Umsetzungsprodukte von 7 bis 8 Mol Ethylenoxid an 1 Mol eines $C_{13}/C_{15}$-Oxoalkohols. Weitere geeignete nichtionische Tenside sind Phenolalkoxylate, wie beispielsweise p-tert.-Butylphenol, das mit 9 Mol Ethylenoxid umgesetzt ist, oder Methylether von Umsetzungsprodukten aus 1 Mol eines $C_{12}$- bis $C_{18}$-Alkohols und 7,5 Mol Ethylenoxid.

Die oben beschriebenen nichtionischen Tenside können beispielsweise durch Veresterung mit Schwefelsäure in die entsprechenden Schwefelsäurehalbester überführt werden. Die Schwefelsäurehalbester werden in Form der Alkalimetall- oder Ammoniumsalze als anionische Tenside eingesetzt. Als anionische Tenside eignen sich beispielsweise Alkalimetall- oder Ammoniumsalze von Schwefelsäurehalbestern von Additionsprodukten von Ethylenoxid und/oder Propylenoxid an Fettalkohole, Alkalimetall- oder Ammoniumsalze von Alkylbenzolsulfonsäure oder von Alkylphenolethersulfaten. Produkte der genannten Art sind im Handel erhältlich. Beispielsweise sind das Natriumsalz eines Schwefelsäurehalbesters eines mit 106 Mol Ethylenoxid umgesetzten $C_{13}/C_{15}$-Oxoalkohols, das Triethanolaminsalz von Dodecylbenzolsulfonsäure, das Natriumsalz von Alkylphenolethersulfaten und das Natriumsalz des Schwefelsäurehalbesters eines Umsetzungsprodukts von 106 Mol Ethylenoxid mit 1 Mol Talgfettalkohol handelsübliche einsetzbare anionische Tenside. Weitere geeignete anionische Tenside sind Schwefelsäurehalbester von $C_{13}/C_{15}$-Oxoalkoholen, Paraffinsulfonsäuren, wie $C_{15}$-Alkylsulfonat, alkylsubstituierte Benzolsulfonsäuren und alkylsubstituierte Naphthalinsulfonsäuren wie Dodecylbenzolsulfonsäure und Di-n-butylnaphthalinsulfonsäure, sowie Fettalkoholphosphate, wie $C_{15}/C_{18}$-Fettalkoholphosphat. Die polymerisierbare wässrige Mischung kann Kombinationen aus einem nichtionischen Tensid und einem anionischen Tensid oder Kombinationen aus nichtionischen Tensiden oder Kombinationen aus anionischen Tensiden enthalten. Auch kationische Tenside sind geeignet. Beispiele hierfür sind die mit Dimethylsulfat quaternierten Umsetzungsprodukte von 6,5 Mol Ethylenoxid mit 1 Mol Oleylamin, Distearyldimethylammoniumchlorid, Lauryltrimethylammoniumchlorid, Cetylpyridiniumbromid und mit Dimethylsulfat quaternierter Stearinsäuretriethanolaminester, der bevorzugt als kationisches Tensid eingesetzt wird.

Der Tensidgehalt, bezogen auf das unneutralisierte Monomer a) beträgt vorzugsweise 0,01 bis 10 Gew.-%, besonders bevorzugt 0,1 bis 5 Gew.-%, ganz besonders bevorzugt 0,5 bis 3 Gew.-%.

Mit den ethylenisch ungesättigten, säuregruppentragenden Monomeren a) copolymerisierbare ethylenisch ungesättigte Monomere e) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Lösevermittler f) sind mit Wasser mischbare organische Lösemittel, beispielsweise Dimethylsulfoxid, Dimethylformamid, N-Methylpyrrolidon, einwertige Alkohole, Glykole, Polyethylenglykole bzw. davon abgeleitete Monoether, wobei die Monoether keine Doppelbindungen im Molekül enthalten. Geeignete Ether sind Methylglykol, Butylglykol, Butyldiglykol, Methyldiglykol, Butyltriglykol, 3-Ethoxy-1-propanol und Glyzerinmonomethylether.

Falls Lösevermittler f) eingesetzt werden, beträgt ihr Gehalt in der Monomerlösung oder -suspension vorzugsweise bis zu 50 Gew.-%, besonders bevorzugt 1 bis 25 Gew.-%, ganz besonders bevorzugt 5 bis 10 Gew.-%.

Die Monomerlösung oder -suspension kann Verdicker, Schaumstabilisatoren, Füllstoffe, Fasern und/oder Zellkeimbildner g) enthalten. Verdicker werden beispielsweise zur Optimierung der Schaumstruktur und zur Verbesserung der Schaumstabilität eingesetzt. Man erreicht damit, dass der Schaum während der Polymerisation nur geringfügig schrumpft. Als Verdickungsmittel kommen alle hierfür bekannten natürlichen und synthetischen Polymeren in Betracht, die die Viskosität eines wässrigen Systems stark erhöhen und nicht mit den Aminogruppen der basischen Polymeren reagieren. Hierbei kann es sich um wasserquellbare oder wasserlösliche synthetische und natürliche Polymere handeln. Eine ausführliche Übersicht über Verdicker findet man beispielsweise in den Veröffentlichungen von R.Y. Lochhead und W.R. Fron, Cosmetics & Toiletries, 108, 95-135 (Mai 1993) und M.T. Clarke, "Rheological Additives" in D. Laba (ed.) "Rheological Properties of Cosmetics and Toiletries", Cosmetic Science and Technology Series, Vol. 13, Marcel Dekker Inc., New York 1993.

Als Verdicker in Betracht kommende wasserquellbare oder wasserlösliche synthetische Polymere sind beispielsweise hochmolekulare Polyethylenglykole oder Copolymerisate aus Ethylenglykol und Propylenglykol sowie hochmolekulare Polysaccharide, wie Stärke, Guarkernmehl, Johannisbrotkernmehl oder Derivate von Naturstoffen, wie Carboxymethylcellulose, Hydroxyethylcellulose, Hydroxymethylcellulose, Hydroxypropylcellulose und Cellulosemischether. Eine weitere Gruppe von Verdickern sind wasserunlösliche Produkte, wie feinteiliges Siliciumdioxid, Zeolithe, Bentonit, Cellulosepulver oder andere feinteilige Pulver von vernetzten Polymerisaten. Die Monomerlösung oder -suspension kann die Verdicker in Mengen bis zu 30 Gew.-% enthalten. Falls solche Verdickungsmittel überhaupt eingesetzt werden, sind sie in Mengen von 0,1 bis 10 Gew.-%, vorzugsweise 0,5 bis 20 Gew.-% in der Monomerlösung odersuspension enthalten.

Um die Schaumstruktur zu optimieren, kann man wahlweise Kohlenwasserstoffe mit mindestens 5 C-Atomen im Molekül zu der wässrigen Reaktionsmischung zusetzen. Geeignete Kohlenwasserstoffe sind beispielsweise Pentan, Cyclopentan, Hexan, Cyclohexan, Heptan, Octan, Isooctan, Decan und Dodecan. Die in Betracht kommenden aliphatischen Kohlenwasserstoffe können geradkettig, verzweigt oder zyklisch sein und haben eine Siedetemperatur, die oberhalb der Temperatur der wässrigen Mischung während des Schäumens liegt. Die aliphatischen Kohlenwasserstoffe erhöhen die Standzeit der noch nicht polymerisierten geschäumten wässrigen Reaktionsmischung. Dadurch wird das Handling der noch nicht polymerisierten Schäume erleichtert und die Prozesssicherheit erhöht. Die Kohlenwasserstoffe wirken beispielsweise als Zellkeimbildner und stabilisieren gleichzeitig den bereits gebildeten Schaum. Darüber hinaus können sie beim Polymerisieren der Monomerlösung oder -suspension ein weiteres Schäumen bewirken. Sie können dann auch die Funktion eines Treibmittels haben. Anstelle von Kohlenwasserstoffen oder in Mischung damit kann man wahlweise auch chlorierte oder fluorierte Kohlenwasserstoffe als Zellkeimbildner und/oder Schaumstabilisator einsetzen, wie Dichlormethan, Trichlormethan, 1,2-Dichlorethan, Trichlorfluormethan oder 1,1,2-Trichlortrifluorethan. Falls Kohlenwasserstoffe eingesetzt werden, verwendet man sie beispielsweise in Mengen von 0,1 bis 20 Gew.-%, vorzugsweise 0,1 bis 10 Gew.-%, bezogen auf die Monomerlösung odersuspension.

Um die Eigenschaften der Schaumstoffe zu modifizieren, kann man einen oder mehrere Füllstoffe zusetzen, beispielsweise Kreide, Talkum, Ton ("Clay"), Titandioxid, Magnesiumoxid, Aluminiumoxid, Fällungskieselsäuren in hydrophilen oder hydrophoben Modifikationen, Dolomit und/oder Kalziumsulfat. Die Füllstoffe können in Mengen bis zu 30 Gew.-% in der Monomerlösung oder -suspension enthalten sein.

Die oben beschriebenen wässrigen Monomerlösungen oder -suspensionen werden zunächst geschäumt. Man kann beispielsweise ein inertes Gas, wie Stickstoff, Kohlendioxid oder Luft, unter einem Druck von beispielsweise 2 bis 400 bar in der wässrigen Monomerlösung oder -suspension lösen und sie anschließend auf Atmosphärendruck entspannen. Beim Entspannen aus mindestens einer Düse entsteht ein fließfähiger Monomerschaum. Da die Gaslöslichkeit mit fallender Temperatur zunimmt, sollte die Gassättigung und das anschließende Schäumen bei möglichst niedriger Temperatur durchgeführt werden, wobei unerwünschte Ausfällungen vermieden werden sollten. Man kann die wässrigen Monomerlösungen oder -suspensionen auch nach einer anderen Methode schäumen, indem man darin feine Blasen eines inerten Gases dispergiert. Das Schäumen der wässrigen Monomerlösungen oder -suspensionen kann im Labor beispielsweise dadurch erfolgen, dass man die wässrige Monomerlösung oder -suspension in einer Küchenmaschine, die mit einem Schneebesen ausgerüstet ist, schäumt. Weiterhin ist es möglich die wässrigen Monomerlösungen oder -suspensionen mit Kohlendioxid zu Schäumen, indem zur Neutralisation Karbonate oder Hydrogenkarbonate eingesetzt werden.

Die Schaumerzeugung wird vorzugsweise in einer Inertgasatmosphäre und mit Inertgasen durchgeführt, beispielsweise durch Versetzen mit Stickstoff oder Edelgasen unter Normaldruck oder erhöhtem Druck, beispielsweise bis zu 25 bar und anschließendes Entspannen. Die Konsistenz der Monomerschäume, die Größe der Gasblasen und die Verteilung der Gasblasen im Monomerschaum kann beispielsweise durch die Auswahl der Tenside d), Lösevermittler f), Schaumstabilisatoren, Zellkeimbildner, Verdickungsmittel und Füllstoffe g) in einem weiten Bereich variiert werden. Dadurch kann man die Dichte, den Grad der Offenzelligkeit und die Wandstärke des Monomerschaums leicht einstellen. Die wässrige Monomerlösung oder suspension wird vorzugsweise bei Temperaturen geschäumt, die unterhalb des Siedepunkts ihrer Bestandteile liegen, beispielsweise bei Umgebungstemperatur bis zu 100°C, vorzugsweise bei 0 bis 50°C, besonders bevorzugt bei 5 bis 20°C. Man kann jedoch auch bei Temperaturen oberhalb des Siedepunkts der Komponente mit dem niedrigsten Siedepunkt arbeiten, indem man die wässrige Monomerlösung oder -suspension in einem druckdicht verschlossenen Behälter schäumt. Man erhält Monomerschäume, die fließfähig und über einen längeren Zeitraum stabil sind. Die Dichte der Monomerschäume beträgt bei einer Temperatur von 20°C beispielsweise 0,01 bis 0,9 g/cm$^3$.

Der erhaltene Monomerschaum kann auf einer geeigneten Unterlage polymerisiert werden. Die Polymerisation wird in Gegenwart üblicher Radikale bildender Initiatoren c) durchgeführt. Die Radikale können beispielsweise durch Erwärmen (thermische Polymerisation) oder durch Bestrahlung mit Licht einer geeigneten Wellenlänge (UV-Polymerisation) erzeugt werden.

Polymere Schäume mit einer Schichtdicke von bis zu etwa 5 Millimeter stellt man beispielsweise durch einseitiges oder beidseitiges Erwärmen oder insbesondere durch einseitiges oder beidseitiges Bestrahlen der Monomerschäume her. Falls dickere polymere Schäume hergestellt werden sollen, beispielsweise polymere Schäume mit Dicken von mehreren Zentimetern, ist die Erwärmung des Monomerschaums mit Hilfe von Mikrowellen besonders vorteilhaft, weil auf diesem Wege eine relativ gleichmäßige Erwärmung erreicht werden kann. Mit zunehmender Schichtdicke nimmt aber der Anteil an nicht umgesetzten Monomer a) und Vernetzer b) im erhaltenen polymeren Schaum zu. Die thermische Polymerisation erfolgt dabei beispielsweise bei Temperaturen von 20 bis 180°C, vorzugsweise in dem Bereich von 40°C bis 160°C, insbesondere bei Temperaturen von 65 bis 140°C. Bei dickeren polymeren Schäumen kann der Monomerschaum beidflächig erwärmt und/oder bestrahlt werden, beispielsweise mit Hilfe einer Kontaktheizung oder durch Bestrahlung oder in einem Trockenschrank. Die erhaltenen polymeren Schäume sind offenzellig. Der Anteil an offenen Zellen beträgt beispielsweise mindestens 80%, vorzugsweise liegt er oberhalb von 90%. Besonders bevorzugt sind polymere Schäume mit einem offenzelligen Anteil von 100%. Der Anteil an offenen Zellen im polymeren Schaum wird beispielsweise mit Hilfe der Rasterelektronenmikroskopie (Scanning Electron Microscopy) bestimmt.

Nach dem Polymerisieren des Monomerschaums oder während des Polymerisierens erfolgt die Trocknung des polymeren Schaums. Hierbei werden Wasser und andere flüchtige Bestandteile entfernt. Beispiele für geeignete Trocknungsverfahren sind thermische Konvektionstrocknung, wie Umlufttrocknung, thermische Kontakttrocknung, wie Walzentrocknung, Strahlungstrocknung, wie Infrarottrocknung, dielektrische Trocknung, wie Mikrowellentrocknung, und Gefriertrocknung.

Die Trocknungstemperaturen liegen üblicherweise im Bereich 50 bis 250°C, vorzugsweise 70 bis 200°C, besonders bevorzugt 90 bis 170°C, ganz besonders bevorzugt 100 bis 150°C. Die bevorzugte Verweilzeit bei dieser Temperatur im Trockner beträgt vorzugsweise 1 bis 60 Minuten, besonders bevorzugt 2 bis 30 Minuten, ganz besonders bevorzugt 5 bis 15 Minuten.

Um unerwünschte Zersetzungs- und Vernetzungsreaktionen zu vermeiden, kann es vorteilhaft sein, die Trocknung bei reduziertem Druck, unter einer Schutzgasatmosphäre und/oder unter schonenden thermischen Bedingungen, bei denen die Produkttemperatur 120°C, bevorzugt 100°C, nicht überschreitet, durchzuführen. Ein besonders geeignetes Trocknungsverfahren stellt die (Vakuum)bandtrocknung dar.

Nach dem Trocknungsschritt enthält der polymere Schaum meistens weniger als 10 Gew.-% Wasser. Der Wassergehalt des polymeren Schaums kann jedoch durch Befeuchten mit Wasser oder Wasserdampf beliebig eingestellt werden.

Der getrocknete polymere Schaum wird hiernach gemahlen und klassiert, wobei zur Mahlung üblicherweise ein- oder mehrstufige Walzenstühle, Stiftmühlen, Hammermühlen oder Schwingmühlen eingesetzt werden können. In einer bevorzugten Ausführungsform wird der getrocknete polymere Schaum mittels einer Schneidmühle vorgemahlen und anschließend mittels einer Pralltellermühle nachgemahlen.

Vorteilhaft wird ein vorgetrockneter polymerer Schaum mit einem Wassergehalt von 5 bis 30 Gew.-%, besonders bevorzugt von 8 bis 25 Gew.-%, ganz besonders bevorzugt von 10 bis 20 Gew.-%, gemahlen und auf den gewünschten Endwassergehalt nachgetrocknet. Die Mahlung eines nur vorgetrockneten polymeren Schaums führt zu weniger unerwünscht kleinen Polymerpartikeln.

Die wasserabsorbierenden Polymerpartikel werden unter Verwendung entsprechender Siebe auf eine Partikelgröße im Bereich von vorzugsweise 100 bis 1.000 $\mu$m, besonders bevorzugt 150 bis 850 $\mu$m, ganz besonders bevorzugt von 150 bis 600 $\mu$m, abgesiebt.

Die mittlere Partikelgröße der als Produktfraktion abgetrennten Polymerpartikel beträgt vorzugsweise mindestens 200 $\mu$m, besonders bevorzugt von 250 bis 600 $\mu$m, ganz besonders von 300 bis 500 $\mu$m. Die mittlere Partikelgröße der Produktfraktion kann mittels der von der EDANA empfohlenen Testmethode Nr. WSP 220.2-05 "Partikel size distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von mindestens 150 $\mu$m beträgt vorzugsweise mindestens 90 Gew.-%, besonders bevorzugt mindesten 95 Gew.-%, ganz besonders bevorzugt mindestens 98 Gew.-%.

Polymerpartikel mit zu niedriger Partikelgröße senken die Permeabilität (SFC). Daher sollte der Anteil zu kleiner Polymerpartikel (Unterkorn) niedrig sein.

Zu kleine Polymerpartikel werden daher üblicherweise abgetrennt.

Es ist auch möglich in späteren Verfahrensschritten zu kleine Polymerpartikel abzutrennen, beispielsweise nach der Oberflächennachvernetzung oder einem anderen Beschichtungsschritt.

Der Anteil an Partikeln mit einer Partikelgröße von höchstens 850 $\mu$m, beträgt vorzugsweise mindestens 90 Gew.-%, besonders bevorzugt mindesten 95 Gew.-%, ganz besonders bevorzugt mindestens 98 Gew.-%.

Der Anteil an Partikeln mit einer Partikelgröße von höchstens 710 $\mu$m, beträgt vorzugsweise mindestens 90 Gew.-%, besonders bevorzugt mindesten 95 Gew.-%, ganz besonders bevorzugt mindestens 98 Gew.-%.

Der Anteil an Partikeln mit einer Partikelgröße von höchstens 600 $\mu$m, beträgt vorzugsweise mindestens 90 Gew.-%, besonders bevorzugt mindesten 95 Gew.-%, ganz besonders bevorzugt mindestens 98 Gew.-%.

Polymerpartikel mit zu großer Partikelgröße sind weniger mechanisch stabil. Daher sollte der Anteil zu großer Polymerpartikel ebenfalls niedrig sein.

Zu große Polymerpartikel werden daher üblicherweise abgetrennt und in die Mahlung des getrockneten Polymergels rückgeführt.

Die Polymerpartikel können zur weiteren Verbesserung der Eigenschaften oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder ß-Hydroxyalkylamide, wie in DE 102 04 938 A1 und US 6,239,230 beschrieben.

Des weiteren sind in DE 40 20 780 C1 zyklische Karbonate, in DE 198 07 502 A1 2-Oxazolidon und dessen Derivate, wie 2-Hydroxyethyl-2-oxazolidon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidone, in DE 102 04 937 A1 zyklische Harnstoffe, in DE 103 34 584 A1 bizyklische Amidacetale, in EP 1 199 327 A2 Oxetane und zyklische Harnstoffe und in WO 2003/31482 A1 Morpholin-2,3-dion und dessen Derivate als geeignete Oberflächennachvernetzer beschrieben.

Bevorzuge Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyloxazolidin-2-on, Oxazolidin-2-on und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 2 Gew.-%, besonders bevorzugt 0,02 bis 1 Gew.-%, ganz besonders bevorzugt 0,05 bis 0,2 Gew.-%, jeweils bezogen auf die Polymerpartikel.

In einer bevorzugten Ausführungsform werden vor, während oder nach der Oberflächennachvernetzung zusätzlich zu den Oberflächennachvernetzern polyvalente Kationen auf die Partikeloberfläche aufgebracht.

Die einsetzbaren polyvalenten Kationen sind beispielsweise zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat und Lactat, möglich. Aluminiumsulfat ist bevorzugt. Außer Metallsalzen können auch Polyamine als polyvalente Kationen eingesetzt werden.

Die Einsatzmenge an polyvalentem Kation beträgt beispielsweise 0,001 bis 1,5 Gew.-%, vorzugsweise 0,005 bis 1 Gew.-%, besonders bevorzugt 0,02 bis 0,8 Gew.-%. jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit der Oberflächennachvernetzer beschichteten Polymerpartikel thermisch getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt werden. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; DE), Vrieco-Nauta Continuous Mixer (Hosokawa Micron BV; Doetinchem; NL), Processall Mixmill Mixer (Processall Incorporated; Cincinnati; US) und Schugi Flexomix® (Hosokawa Micron BV; Doetinchem; NL). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; DE), Hosokawa Bepex® Disc Dryer (Hosokawa Micron GmbH; Leingarten; DE) und Nara Paddle Dryer (NARA Machinery Europe; Frechen; DE). Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Trocknungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise 10 bis 120 Minuten, besonders bevorzugt 20 bis 90 Minuten, ganz besonders bevorzugt 30 bis 60 Minuten.

Anschließend können die oberflächennachvernetzten Polymerpartikel erneut klassiert werden.

In einer bevorzugten Ausführungsform wird die Oberflächennachvernetzung bereits auf der Stufe des polymeren Schaums durchgeführt, wobei die für die Polymerpartikel genannten Mengen und Temperaturen für den polymeren Schaum entsprechend gelten.

Weiterhin können die Polymerpartikel zur Verbesserung der Eigenschaften beschichtet oder nachbefeuchtet werden.

Die Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität erhöht und die Neigung zur statischen Aufladung vermindert.

Geeignete Beschichtungen zur Verbesserung der Quellgeschwindigkeit (FSR) sowie der Flüssigkeitsweiterleitung (SFC) sind beispielsweise anorganische inerte Substanzen, wie wasserunlösliche Metallsalze, organische Polymere, kationische Polymere sowie zwei- oder mehrwertige Metallkationen, wie Aluminiumsulfat und Aluminuimlaktat. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20. Geeignete Beschichtungen zur Verminderung des Gehaltes an nicht umgesetzten Monomeren (Restmonomere) sind beispielsweise Reduktionsmittel, wie die Salze der schwefeligen Säure, der unterphosphorigen Säure und/oder organischer Sulfinsäure. Als Reduktionsmittel wird aber vorzugsweise ein Gemisch aus dem Natriumsalz der 2-Hydroxy-2-sulfinatoessigsäure, dem Dinatriumsalz der 2-Hydroxy-2-sulfonatoessigsäure und Natriumhydrogensulfit eingesetzt. Derartige Gemische sind als Brüggolite® FF6 und Brüggolite® FF7 (Brüggemann Chemicals; Heilbronn; DE) erhältlich.

In einer bevorzugten Ausführungsform wird die Nachbefeuchtung und/oder die Beschichtung bereits auf der Stufe des polymeren Schaums durchgeführt.

Die wasserabsorbierenden Polymerpartikel weisen einen Feuchtegehalt von vorzugsweise 0 bis 15 Gew.-%,

besonders bevorzugt 0,2 bis 10 Gew.-%, ganz besonders bevorzugt 0,5 bis 8 Gew.-%, auf, wobei der Wassergehalt gemäß der von der EDANA empfohlenen Testmethode Nr. WSP 230.2-05 "Moisture content" bestimmt wird.

Die wasserabsorbierenden Polymerpartikel weisen eine Zentrifugenretentionskapazität (CRC) von typischerweise mindestens 10 g/g, vorzugsweise mindestens 15 g/g, bevorzugt mindestens 20 g/g, besonders bevorzugt mindestens 22 g/g, ganz besonders bevorzugt mindestens 25 g/g, auf. Die Zentrifugenretentionskapazität (CRC) der wasserabsorbierenden Polymerpartikel beträgt üblicherweise weniger als 40 g/g. Die Zentrifugenretentionskapazität (CRC) wird gemäß der von der EDANA empfohlenen Testmethode Nr. WSP 241.2-05 "Centrifuge retention capacity" bestimmt.

Die wasserabsorbierenden Polymerpartikel weisen eine Blutabsorption von typischerweise mindestens 8 g/g, vorzugsweise mindestens 12 g/g, bevorzugt mindestens 15 g/g, besonders bevorzugt mindestens 18 g/g, ganz besonders bevorzugt mindestens 20 g/g, auf. Die Blutabsorption der wasserabsorbierenden Polymerpartikel beträgt üblicherweise weniger als 30 g/g.

Die wasserabsorbierenden Polymerpartikel weisen eine Absorption unter einem Druck von 49,2 $g/cm^2$ (AUL0.7psi) von typischerweise mindestens 10 g/g, vorzugsweise mindestens 13 g/g, bevorzugt mindestens 16 g/g, besonders bevorzugt mindestens 18 g/g, ganz besonders bevorzugt mindestens 20 g/g, auf. Die Absorption unter einem Druck von 49,2 $g/cm^2$ (AUL0.7psi) der wasserabsorbierenden Polymerpartikel beträgt üblicherweise weniger als 30 g/g. Die Absorption unter einem Druck von 49,2 $g/cm^2$ (AUL0.7psi) wird analog der von der EDANA empfohlenen Testmethode Nr. WSP 242.2-05 "Absorption under pressure" bestimmt, wobei statt eines Drucks von 21,0 $g/cm^2$ ein Druck von 49,2 $g/cm^2$ eingestellt wird.

Die erfindungsgemäß zu verwendenden wasserabsorbierenden Polymerpartikel weisen eine hohe Absorptionskapazität für Blut und eine hohe Quellgeschwindigkeit auf und sind daher zum Einsatz in Hygienartikeln zur Absorption von Blut und Menstruationsflüssigkeit besonders geeignet.

Methoden:

Die Messungen sollten, wenn nicht anders angegeben, bei einer Umgebungstemperatur von 23 $\pm$ 2 °C und einer relativen Luftfeuchte von 50 $\pm$ 10 % durchgeführt werden. Die wasserabsorbierenden Polymerpartikel werden vor der Messung gut durchmischt.

Blutabsorption

Die Blutabsorption wird gemäß der von der EDANA empfohlenen Testmethode Nr. WSP 241.2-05 "Centrifuge Retention Capacity" bestimmt, wobei statt einer 0,9 gew.-%igen wässrigen Natriumchloridlösung ein gemäß US 6,147,424 (Spalte 17, Zeile 33 bis Spalte 18, Zeile 45) modifiziertes Schafblut verwendet wird.

Quellgeschwindigkeit (Free Swell Rate)

Zur Bestimmung der Quellgeschwindigkeit (FSR) werden 1,00 g (= W1) wasserabsorbierende Polymerpartikel in ein 25 ml Becherglas eingewogen und gleichmäßig auf dessen Boden verteilt. Dann werden 20 ml einer 0,9 gew.%-igen Kochsalzlösung in ein zweites Becherglas dosiert und der Inhalt dieses Glases wird dem ersten zügig hinzugefügt und eine Stoppuhr gestartet. Sobald der letzte Tropfen der Kochssalzlösung absorbiert wurde, was man am Verschwinden der Reflexion auf der Flüssigkeitsoberfläche erkennt, wird die Stoppuhr angehalten. Die genaue Flüssigkeitsmenge, die aus dem zweiten Becherglas ausgegossen und durch die wasserabsorbierenden Polymerpartikel im ersten Becherglas absorbiert wurde, wird durch Rückwägung des zweiten Becherglases genau bestimmt (=W2). Die für die Absorption benötigte Zeitspanne, die mit der Stoppuhr gemessen wurde, wird als t bezeichnet. Das Verschwinden des letzten Flüssigkeitstropfens auf der Oberfläche wird als Zeitpunkt t bestimmt.

Daraus errechnet sich die Quellgeschwindigkeit (FSR) wie folgt:

$$FSR\ [g/gs] = W2/(W1 x t)$$

Wenn der Feuchtegehalt der wasserabsorbierenden Polymerpartikel mehr als 3 Gew.-% beträgt, so ist das Gewicht W1 um diesen Feuchtegehalt zu korrigieren.

Beispiele

Beispiel 1 (nicht erfindungsgemäß)

81,1 g Acrylsäure, 425,7 g einer 37,3 gew.-%igen wässrigen Natriumacrylatlösung, 3,0 g Sartomer® SR-344 (Diacrylat eines Polyethylenglykols mit einem Molgewicht von ca. 400 g/mol), 12,8 g einer 15 gew.-%igen wässrigen Lösung von Lutensol® AT80 (Additionsprodukt von 80 Mol Ethylenoxid an 1 Mol eines linearen, gesättigten $C_{16}$-$C_{18}$ Fettalkohols; BASF SE; Ludwigshafen; DE), 0,4 g Irgacure® 2959 (1-[4-(2-Hydroxyethoxy)-phenyl]-2-hydroxy-2-methylpropan-1-on) und 77,1 g Wasser wurden in einem Becherglas gemischt.

Die erhaltene homogene Lösung wurde in einen Druckbehälter überführt und dort für 25 Minuten bei einem Druck von 10 bar und einem Durchfluss von 300 l/h mit Kohlendioxid gesättigt. Unter Druck wurden 4,0 g einer 3 gew.-%igen wässrigen Lösung von 2,2'-Azobis(2-amidinopropan)dihydrochlorid zugegeben und mit einem starken Kohlendioxidstrom untergemischt. Anschließend wurde für weitere 5 Minuten Kohlendioxid durch die Reaktionsmischung geleitet. Die mit Kohlendioxid gesättigte Reaktionsmischung wurde danach bei einem Druck von 12 bar durch eine Düse mit einem Durchmesser von 1,0 mm ausgepresst, wobei sich ein feinzelliger, gut fließfähiger Schaum bildete.

Der erhaltene Monomerschaum wurde auf eine DIN A3 große Glasplatte mit 3 mm hohen Rändern aufgebracht und mit einer zweiten Glassplatte bedeckt. Die Schaumprobe wurde synchron von beiden Seiten über 4 Minuten mit UV-Licht bestrahlt, von oben mit einem UV/VIS-Strahler UVASPOT 1000/T (Dr. Hönle AG; Gräfelfing; DE), von unten mit 2 UV/VIS-Strahlern UVASPOT 400/T (Dr. Hönle AG; Gräfelfing; DE).

Die erhaltene Schaumschicht wurde in einem Umlufttrockenschrank bei 100°C vollständig getrocknet, anschließend in einer Retschmühle gemahlen und auf eine Partikelgröße von 150 bis 600 $\mu$m abgesiebt.

| | |
|---|---|
| Feststoffgehalt der Reaktionsmischung: | 40,6 Gew.-% |
| Neutralisationsgrad: | 60 mol-% |

Die erhaltenen wasserabsorbierenden Polymerpartikel hatten eine Blutabsorption von 17,9 g/g und eine Quellgeschwindigkeit von 2,0 g/gs.

Beispiel 2 (Vergleichsbeispiel)

Es wurde verfahren wie unter Beispiel 1. Die Monomerlösung wurde nicht aufgeschäumt. Die erhaltenen wasserabsorbierenden Polymerpartikel hatten eine Blutabsorption von 14,6 g/g und eine Quellgeschwindigkeit von 0,17 g/gs.

Beispiel 3 (nicht erfindungsgemäß)

135,24 g Acrylsäure, 709,82 g einer 37,3 gew.-%igen wässrigen Natriumacrylatlösung, 8,0 g Sartomer® SR-344 (Diacrylat eines Polyethylenglykols mit einem Molgewicht von ca. 400 g/mol), 21,33 g einer 15 gew.-%igen wässrigen Lösung von Lutensol® AT80 (Additionsprodukt von 80 Mol Ethylenoxid an 1 Mol eines linearen, gesättigten $C_{16}$-$C_{18}$ Fettalkohols; BASF SE; Ludwigshafen; DE), 0,333 g Irgacure® 2959 (1-[4-(2-Hydroxyethoxy)-phenyl]-2-hydroxy-2-methylpropan-1-on) und 125,61 g Wasser wurden in einem Becherglas gemischt.

Die erhaltene homogene Lösung wurde in einen Druckbehälter überführt und dort für 25 Minuten bei einem Druck von 12 bar und einem Durchfluss von 300 l/h mit Kohlendioxid gesättigt. Unter Druck wurden 6,67 g einer 3 gew.-%igen, wässrigen Lösung von Wako® V-50 (2,2'-Azo-bis-(2-amidinopropan)-dihydrochlorid) zugegeben und mit einem starken Kohlendioxidstrom untergemischt. Anschließend wurde für weitere 5 Minuten Kohlendioxid durch die Reaktionsmischung geleitet. Die mit Kohlendioxid gesättigte Reaktionsmischung wurde danach bei einem Druck von 12 bar durch eine Düse mit einem Durchmesser von 1,0 mm ausgepresst, wobei sich ein feinzelliger, gut fließfähiger Schaum bildete.

Der Boden einer DIN A3 große Glasplatte mit 3 mm hohen Rändern wurde mit einer durchsichtigen Polyesterfolie bedeckt. Der erhaltene Monomerschaum wurde auf die Glasplatte aufgebracht und mit einer zweiten durchsichtigen Polyesterfolie und einer zweiten Glassplatte bedeckt. Die Schaumprobe wurde synchron von beiden Seiten über 4 Minuten mit UV-Licht bestrahlt, von oben mit einem UV/VIS-Strahler UVASPOT 1000/T (Dr. Hönle AG; Gräfelfing; DE), von unten mit 2 UV/VIS-Strahlern UVASPOT 400/T (Dr. Hönle AG; Gräfelfing; DE).

Der erhaltene polymere Schaum wurde mit 5 gew.-%igen wässrigen Natriummetabisulfit so besprüht dass er anschließend 3 Gew.-% Natriummetabisulfit bezogen auf wasserfreies Polymer enthielt. Das Produkt wurde anschließend 30 Minuten in einem Umlufttrockenschrank bei 100°C getrocknet, anschließend in einer Retschmühle gemahlen und auf eine Partikelgröße von 150 bis 850 $\mu$m abgesiebt.

Feststoffgehalt der Reaktionsmischung: 40,9 Gew.-%
Neutralisationsgrad: 60 mol-%

Die erhaltenen wasserabsorbierenden Polymerpartikel hatten eine Blutabsorption von 16,0 g/g und eine Quellgeschwindigkeit von 2,4 g/gs.

**Patentansprüche**

1. Verwendung wasserabsorbierender Polymerpartikel zur Absorption von Blut und/oder Menstruationsflüssigkeit, wobei die wasserabsorbierenden Polymerpartikel durch Polymerisation einer aufgeschäumten wässrigen Monomerlösung oder -suspension, enthaltend

   a) mindestens ein ethylenisch ungesättigtes, säuregruppentragendes Monomer, das zu 25 bis 95 mol-% neutralisiert ist,
   b) mindestens einen Vernetzer,
   c) mindestens einen Initiator und
   d) mindestens ein Tensid,

   Vortrocknen des polymeren Schaums auf einen Wassergehalt von 5 bis 30 Gew.-%, Mahlung des vorgetrockneten Schaums und Nachtrocknen auf den gewünschten Endwassergehalt erhältlich sind.

2. Verwendung gemäß Anspruch 1, **dadurch gekennzeichnet, dass** mindestens 50 mol-% der neutralisierten Monomere a) mittels einer anorganischen Base neutralisiert wird.

3. Verwendung gemäß Anspruch 2, **dadurch gekennzeichnet, dass** die anorganische Base Kaliumkarbonat, Natriumkarbonat oder Natriumhydroxid ist.

4. Verwendung gemäß einem der Ansprüche 1 bis 3, **dadurch gekennzeichnet, dass** der gemahlene polymere Schaum auf eine Partikelgröße im Bereich von 150 bis 850 $\mu$m klassiert wird.

5. Verwendung gemäß einem der Ansprüche 1 bis 4, **dadurch gekennzeichnet, dass** die Monomerlösung oder -suspension mindestens 1 Gew.-% des Vernetzers b), bezogen auf das unneutralisierte Monomer a), enthält.

6. Verwendung gemäß einem der Ansprüche 1 bis 5, **dadurch gekennzeichnet, dass** das Monomer a) zu mindestens 50 mol-% Acrylsäure ist.

7. Verwendung gemäß einem der Ansprüche 1 bis 6, **dadurch gekennzeichnet, dass** die wasserabsorbierenden Polymerpartikel eine Zentrifugenretentionskapazität von mindestens 10 g/g aufweisen.

8. Verwendung gemäß einem der Ansprüche 1 bis 7, **dadurch gekennzeichnet, dass** die wasserabsorbierenden Polymerpartikel eine Blutabsorption von mindestens 15 g/g aufweisen.

**Claims**

1. The use of water-absorbing polymer particles for absorbing blood and/or menses, the water-absorbing polymer particles being obtainable by polymerizing a foamed aqueous monomer solution or suspension comprising

   a) at least one ethylenically unsaturated monomer which bears acid groups and has been neutralized to an extent of 25 to 95 mol%,
   b) at least one crosslinker,
   c) at least one initiator and
   d) at least one surfactant,

   predrying the polymeric foam to a water content of 5 to 30% by weight, grinding the predried foam and subsequent drying to the desired final water content.

2. The use according to claim 1, wherein at least 50 mol% of the neutralized monomers a) is neutralized by means of an inorganic base.

3. The use according to claim 2, wherein the inorganic base is potassium carbonate, sodium carbonate or sodium hydroxide.

4. The use according to any of claims 1 to 3, wherein the ground polymeric foam is classified to a particle size in the range from 150 to 850 $\mu$m.

5. The use according to any of claims 1 to 4, wherein the monomer solution or suspension comprises at least 1% by weight of the crosslinker b), based on the unneutralized monomer a).

6. The use according to any of claims 1 to 5, wherein the monomer a) is acrylic acid to an extent of at least 50 mol%.

7. The use according to any of claims 1 to 6, wherein the water-absorbing polymer particles have a centrifuge retention capacity of at least 10 g/g.

8. The use according to any of claims 1 to 7, wherein the water-absorbing polymer particles have a blood absorbency of at least 15 g/g.

**Revendications**

1. Utilisation de particules polymères absorbant l'eau pour l'absorption de sang et/ou de liquide de menstruation, les particules polymères absorbant l'eau pouvant être obtenues par polymérisation d'une solution ou d'une suspension aqueuse, moussée, de monomères, contenant

a) au moins un monomère éthyléniquement insaturé, portant des groupes acides, qui est neutralisé à raison de 25 à 95% en mole,
b) au moins un réticulant,
c) au moins un initiateur et
d) au moins un tensioactif,

préséchage de la mousse polymère à une teneur en eau de 5 à 30% en poids, broyage de la mousse préséchée et postséchage à la teneur finale en eau souhaitée.

2. Utilisation selon la revendication 1, **caractérisée en ce qu'**au moins 50% en mole des monomères neutralisés a) sont neutralisés au moyen d'une base inorganique.

3. Utilisation selon la revendication 2, **caractérisée en ce que** la base inorganique est le carbonate de potassium, le carbonate de sodium ou l'hydroxyde de sodium.

4. Utilisation selon l'une quelconque des revendications 1 à 3, **caractérisée en ce que** la mousse polymère moussée est classifiée à une grosseur de particule dans la plage de 150 à 850 $\mu$m.

5. Utilisation selon l'une quelconque des revendications 1 à 4, **caractérisée en ce que** la solution ou la suspension de monomères contient au moins 1% en poids de réticulant b), par rapport au monomère a) non neutralisé.

6. Utilisation selon l'une quelconque des revendications 1 à 5, **caractérisée en ce que** le monomère a) est, à raison d'au moins 50% en mole, de l'acide acrylique.

7. Utilisation selon l'une quelconque des revendications 1 à 6, **caractérisée en ce que** les particules polymères absorbant l'eau présentent une capacité de rétention dans une centrifugeuse d'au moins 10 g/g.

8. Utilisation selon l'une quelconque des revendications 1 à 7, **caractérisée en ce que** les particules polymères absorbant l'eau présentent une absorption du sang d'au moins 15 g/g.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0858478 B1
- WO 9731971 A1
- WO 9944648 A1
- WO 0052087 A1
- WO 2005042042 A1
- WO 2002055469 A1
- WO 2003078378 A1
- WO 2004035514 A1
- US 6107358 A
- EP 0744435 A
- EP 0530438 A1
- EP 0547847 A1
- EP 0559476 A1
- EP 0632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 2002032962 A2
- EP 0083022 A2
- EP 0543303 A1
- EP 0937736 A2
- DE 3314019 A1
- DE 3523617 A1
- EP 0450922 A2
- DE 10204938 A1
- US 6239230 B
- DE 4020780 C1
- DE 19807502 A1
- DE 19807992 C1
- DE 19854573 A1
- DE 19854574 A1
- DE 10204937 A1
- DE 10334584 A1
- EP 1199327 A2
- WO 200331482 A1
- DE 3713601 A1
- US 6147424 A

**In der Beschreibung aufgeführte Nicht-Patentliteratur**

- **F.L. BUCHHOLZ ; A.T. GRAHAM.** Modern Superabsorbent Polymer Technology. Wiley-VCH, 1998, 71-103
- **R.Y. LOCHHEAD ; W.R. FRON.** *Cosmetics & Toiletries,* Mai 1993, vol. 108, 95-135
- **M.T. CLARKE.** Rheological Additives
- Rheological Properties of Cosmetics and Toiletries. Cosmetic Science and Technology Series. Marcel Dekker Inc, 1993, vol. 13